# EUROPEAN PATENT APPLICATION

(11) **EP 4 160 618 A1**
(43) Date of publication of application: **05.04.2023**
(21) Application number: 22199417.1
(22) Date of filing: 03.10.2022
(51) Int. Cl.: G16H 50/30, G16H 20/30

(54) **EXERCISE METRIC ALGORITHM AND SYSTEM FOR CALCULATING AN EXERCISE METRIC**

(30) Priority: 01.10.2021 GB 202114107
(71) Applicant: Aria Code Limited, Bromley BR2 9QQ (GB)
(72) Inventor: LAVILLE, Jay, Bromley, BR2 9QQ (GB)
(74) Representative: Sanger, Phillip Simon

(57) **Abstract**

A computer-implemented method comprising the steps:
collecting a first type of health data over *n* days;
collecting a second type of health data over *m* days;
after n days:
calculating the average of the first type of health data and normalising the average to produce a first sub-score;
updating an aggregate health score with the first sub-score;

after *m* days:
calculating the average of the second type of health data and normalising the average to produce a second sub-score;
updating the aggregate health score with the second sub-score;

repeating the above process by varying n and m at each instance, in which n and m are each selected at random.

## Description

### Technical Field

The present invention is concerned with an algorithm that calculates a combined fitness metric, as well as a system programmed for calculating such a metric.

### Background Art

It is well known in the art of health and fitness equipment and 'trackers' to provide a user with a straightforward fitness metric, usually in combination with a target to be reached over a predetermined amount of time. For example steps taken, or kcal consumed (i.e. burned through exercise) per day. Such metrics may be 'simple'(such as a steps or kcal) or combined. Combined metrics attribute scores to the simple metrics and combine them to provide an overall score. Typically, this involves weighting or normalising the simple metrics.

A technical problem associated with such methods is in the periodic update of the combined metric. Prior art systems sample each of the simple, constituent metrics at predetermined time intervals.

For example, a fitness algorithm may attribute a score out of 100 points for up to 12,000 steps on average per day. So 6,000 steps per day, on average, would score 50 points for the month. The score is calculated (or refreshed) at the end of each month. Therefore the month's score is calculated by summing the number of steps over the month, and dividing by the number of days. The problem with this approach is that it quickly becomes apparent to the user when the score is refreshed, and the score can be influenced by significantly increased work rate on the last few days of the month. So instead of putting in a continuous effort, the algorithm can lead the user to be relatively sedentary for most of the month, with a burst of activity towards the end. This is problematic because exercise is best taken in a consistent manner, rather than 'binged' at the end of a month. Such activity can lead to injury and would not increase the user's general fitness as much as a continuous effort.

This effect is compounded when using combined metrics- for example the score may be made up of both steps and kcal. If the user is aware that the score is calculated at month end, they would be tempted to try and 'up the ante' for the last few days in the month to increase their score.

A further technical problem with known fitness scoring systems is how comparisons with other users is carried out. Typically, systems have thousands of users, all of whom have different scores. Comparing an individual to every other user in the system is not particularly useful- being ranked, say, 4,789^{th} out of several thousand users is not much use. What would be more useful is for a user to be compared to a smaller set (a subset) of users in his or her direct demographic- for example based on their lifestyle. This is inherently difficult without requesting that the user input a significant amount of personal information into the system, which they may be reluctant to do.

Further, there is an inherent advantage in the collection of (anonymised) location-based health data. Determining demographic data of users within a specific area/town who are health conscious can indicate opportunities for gyms or fitness brands.

It is an aim of the present invention to overcome, or at least mitigate, the above problem.

### Summary of Invention

According to a first aspect of the present invention there is provided a computer-implemented method comprising the steps of:
collecting a first type of health data over *n* days;
collecting a second type of health data over *m* days;
after *n* days:
   calculating the average of the first type of health data and normalising the average to produce a first sub-score;
   updating an aggregate health score with the first sub-score;
after *m* days:
   calculating the average of the second type of health data and normalising the average to produce a second sub-score;
   updating the aggregate health score with the second sub-score;
repeating the above steps over a plurality of cycles;
varying n and m between at least two cycles.

Preferably n and m are varied at each cycle.

Preferably n and m are random integers.

Preferably n and m are provided with at least one of an upper and lower limit.

Preferably the first and second types of health data are selected from:
a) energy consumed ("burned");
b) active time;
c) step count; and,
d) power output.

Preferably the method comprises the steps of:
collecting a third type of health data over o days;
calculating the average of the third type of health data and normalising the average to produce a third sub-score;
updating the aggregate health score with the third sub-score;
repeating the above steps over a plurality of cycles;
varying o between at least two cycles.

Preferably the method comprises the steps of:
collecting a fourth type of health data over *p* days;
calculating the average of the third type of health data and normalising the average to produce a fourth sub-score;
updating the aggregate health score with the fourth sub-score;
repeating the above steps over a plurality of cycles;
varying *p* between at least two cycles.

According to a second aspect there is provided a system for determining a user health score comprising:
a first fitness sensor configured to collect a first type of health data;
a second fitness sensor configured to collect a second type of health data;
a computer configured for communication with the first and second fitness sensors, the computer comprising software configured to implement the method of the first aspect.

Preferably the first and second fitness sensors are selected from:
a) energy consumption sensor;
b) active timer;
c) step counter; and,
d) power output meter.

Preferably the power output meter is comprised within an item of weight training equipment.

According to a third aspect there is provided a computer-implemented method comprising the steps of:
providing a first user device associated with a first user, the first user device storing a first user health score;
providing a second user device associated with a second user, the first user device storing a second user health score;
detecting an encounter with the first user device by the presence of the second user device;
anonymising the second user health score;
sending the second user health score from the second user device to the first user device;
comparing the second user health score with the first user health score on the first user device.

Advantageously, user is compared to a smaller set (a subset) of users in his or her direct demographic.

Advantageously, by collecting of (anonymised) location-based health data, commercial opportunities for gyms or fitness brands (such as retail stores) can be determined.

Preferably the method comprises the steps of detecting a plurality of encounters over a predetermined time period to determine a sample, and calculating a rank of the first user health score within the sample.

Preferably the presence of the second user device in the encounter is determined by distance from the first user device, which distance is detected by wireless communication means.

Preferably the wireless communication means is Bluetooth (RTM).

### Brief Description of Drawings

An embodiment of the present invention will now be described with reference to the following figure in which:
FIGURE 1a is a first schematic view of a user of the system of the present invention undertaking a first activity;
FIGURE 1b is a second schematic view of a user of the system of the present invention undertaking a second activity;
FIGURE 2 is a flow chart of a method in accordance with the present invention;
FIGURE 3 is a schematic diagram of how a combined fitness score is updated by the present invention;
FIGURE 4 is a schematic diagram of user activity according to the present invention.

### Description of the first embodiment

### Hardware

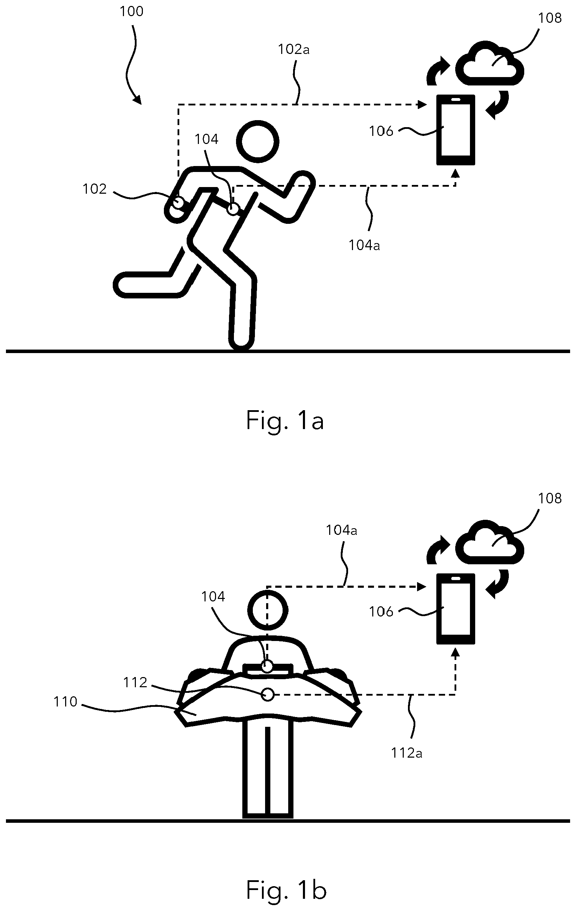

Referring to Figure 1a, a system user 100 is shown utilising a plurality of fitness telemetry devices. In Figure 1a, the user 100 is shown running. The devices are a smart watch 102 and a chest strap heart rate monitor 104. The smart watch 102 is provided with an accelerometer and configured to measure the number of steps the user 100 is taking when the watch 102 is worn. The smart watch 100 may also be configured to record specific exercises (such as running), and may be configured to measure e.g., heat rate (in which case the strap 104 is not required). The chest strap HRM 104 is configured to measure the user's heart rate. Both smart watches 102 and HRMs 104 are well known in the art.

Both the watch 102 and HRM 104 are configured to communicate wirelessly with a user device 106. Wireless data links 102a and 104a are shown in Figure 1a. The user device 106 may be any kind of computer configured to carry out the functions required below, and therefore comprises at least a memory, processor, user input means and display, but otherwise may be e.g. a mobile phone, PDA, tablet computer, PC or similar. The user device 106 also has connectivity with the watch 102 and HRM 104 (which may be via a wireless link such as Bluetooth^{™} or Wi-Fi^{™}) or a wired link such as via a USB cable. The user device 106 is configured for communication with the internet and thereby a cloud server 108 which is owned and managed by a service provider.

The device 106 may be carried with the user- i.e. kept in wireless range of the fitness telemetry devices during exercise, or may be updated with the data from the devices after exercise is carried out, and once the devices have been brought into range.

In Figure 1b, the user 100 is shown undertaking a different kind of fitness activity-weight training. The user is holding a weighted training device 110. The weighted training device 110 comprises a telemetry sensor 112 in the form of an accelerometer that is configured for communication (wireless or wired) with the user device 106. The sensor 112 is configured to determine the number of reps the user carries out. The device may be of the type described in applicant's co-pending GB application 2107632.8.

In the above examples, various types of fitness metrics are measured. These are:
a) Step count from the smartwatch 102 using accelerometers;
b) Heart rate from the HRM 104; and,
c) Mass and number of reps from the sensor 112 in the device 110.

In addition, further inputs may be provided either from telemetry devices as described above, or manually by the user into the device 116. One example is sleep time (may be measured by the smartwatch 102).

In some cases, the device 106 and / or the telemetry devices may process the data. For example, the data from the HRM may be used to calculate active kcal burned during a workout, or, for example, time spent in various heart rate zones.

### Software

The system of the present invention features software configured to calculate a combined score, or meoscore^{™} from several individual measurements or metrics.

In one embodiment, the user's meoscore^{™} is calculated out of 800, and includes the following activity types:
a) Active kcal - a sub-score out of 200 for average kcal burned per day over a given multi-day period. The target is based on various factors such as height, mass, age, gender and fitness.
b) Active time - a sub-score out of 200 for average time spent in high heart rate zones (zones 3 to 5) per day over a given multi-day period. The target is based on various factors such as age, gender which are used to calculate HR bands 1 to 5.
c) Daily steps - a sub-score out of 200 for average steps per day over a given multi-day period. The target is based on various factors such as height, mass, age, gender and fitness.
d) Power - a sub-score out of 200 for force or power output undertaking strength exercises with equipment such as the device 110. The target is based on various factors such as height, mass, age, gender and fitness.

The sub-scores are added to reach the final meoscore^{™} out of 800.

In the present embodiment, the meoscore^{™} is calculated by the device 106 and communicated to the cloud server 108 for backup and comparison purposes. Therefore the use of a loud server 108 is not essential in this embodiment. In an alternative embodiment, the meoscore^{™} may be calculated by the cloud server 108 after receiving data from the device 106, and the meoscore^{™} communicated back to the device to view.

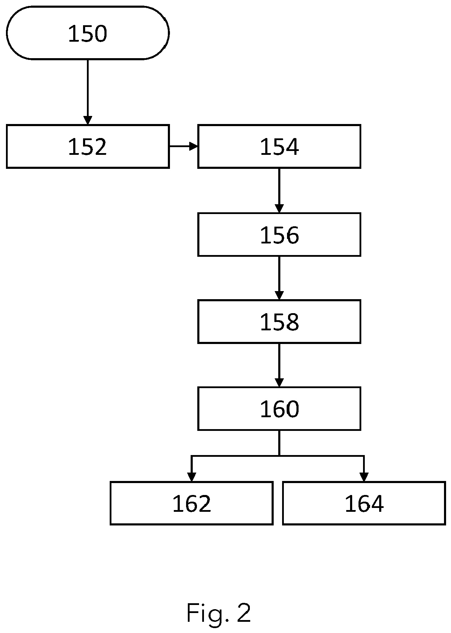

Referring to Figure 2, there is shown a method for caculating the meoscore^{™}. At step 150, the system is initialised. At step 152 the user carries out a monitored activity, which may be a workout (e.g. using a HRM 104) or may simply be walking. The activity generates data at step 154. The data is collected by one or more of the fitness telemetry devices 102, 104, 112. At step 156 the data is transmitted to the user device 106.

Several sets of data are transmitted to the user device 106. These are typically several instances of workouts, sleep, steps etc. The device collects the data over a multi-day period.

At step 158, following a predetermined time interval (discussed below), the user device 106 converts the collected data from at least one of the activities into a sub-score (as discussed above). At step 160, the sub-scores are summed to create the total meoscore^{™} out of 800, which is shared with the user 100 and the cloud server at steps 162, 164 respectively.

As mentioned in the background, known combined metrics utilise a set timeframe for calculation. For example, all of the above individual sub-scores will be calculated on the same day (for example the last day of the calendar month) in relation to the preceding month's activity.

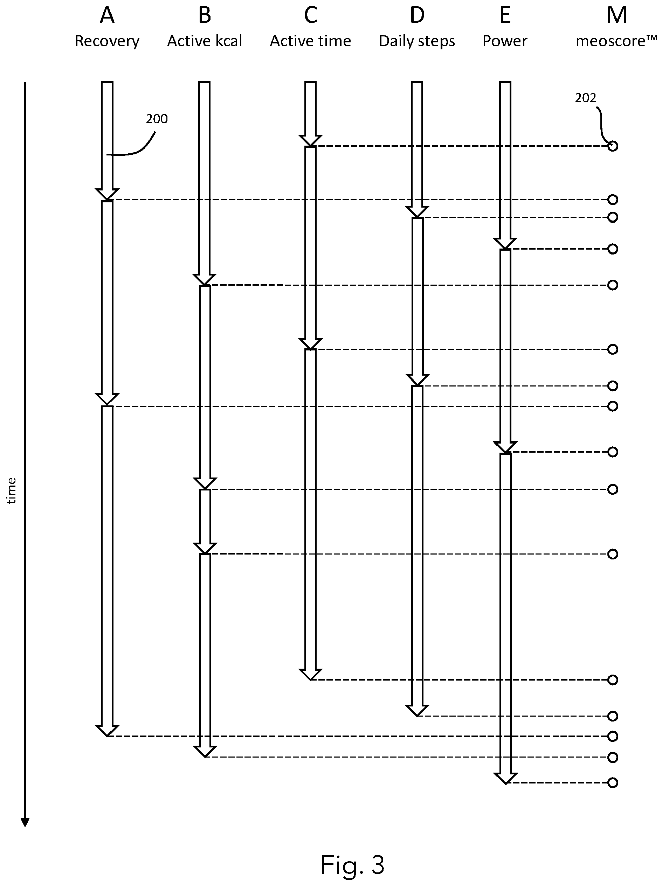

The present invention provides a different approach known as AVRR (algorithmic variable refresh rate). The software on the device 106 is programmed to refresh each sub-score, and hence the total meoscore^{™} (steps 158 / 160) at random intervals. This is explained with reference to Figure 3. Each sub-score A, B, C, D is provided across the top of the diagram (x-axis). Arrows 200 represent the multi-day time periods (intervals) over which the sub-score is calculated. As each sub-score is calculated, the meoscore^{™} is also updated at update points 202.

It will be noted that the device 106 is used to generate a random duration for each interval. Each interval is measured in complete days (i.e. no part-days). The device is provided with a minimum and maximum interval length. This may be, for example, a minimum of 7 days and a maximum of 30 days. It will be noted that the window may be quite small- for example between 25 and 30 days- for the update frequency to be sufficiently random.

The selection of a random number is known in the art and will not be described in detail here.

In this way, there is no predictability to the update frequency, and the user is unable to determine when the next update will occur, preventing 'workout binging'.

### Use

Although the meoscore^{™} is a useful metric for general physical wellbeing, it may also be employed in a number of ways.

One such way is to offer rewards to the user for maintaining a high meoscore^{™}. In one embodiment, the user may receive store discounts or monetary incentives for maintaining a high meoscore^{™}. For example, their gym membership may be discounted. Advantageously, the meoscore^{™} can be transmitted to the gym or store by the cloud server 108. The use of AVRR as described above overcomes any concerns the gym or store may have with abuse of the system and solves the aforementioned technical problems with existing health metrics. The user needs to maintain a constant fitness regime to ensure that their meoscore^{™} remains high.

### Description of the second embodiment

According to a second embodiment of the invention, the user's meoscore^{™} (or other fitness score or metric) is calculated as described herein. This is then stored on the user device 106 (which, as described above may also be combined with the smartwatch 102).

The device 106 is equipped with short-range wireless communication functionality, such as Bluetooth^{™}. Therefore the device 106 is capable of detecting other user devices in its vicinity (within a predetermined range of, say 0 - 30m).

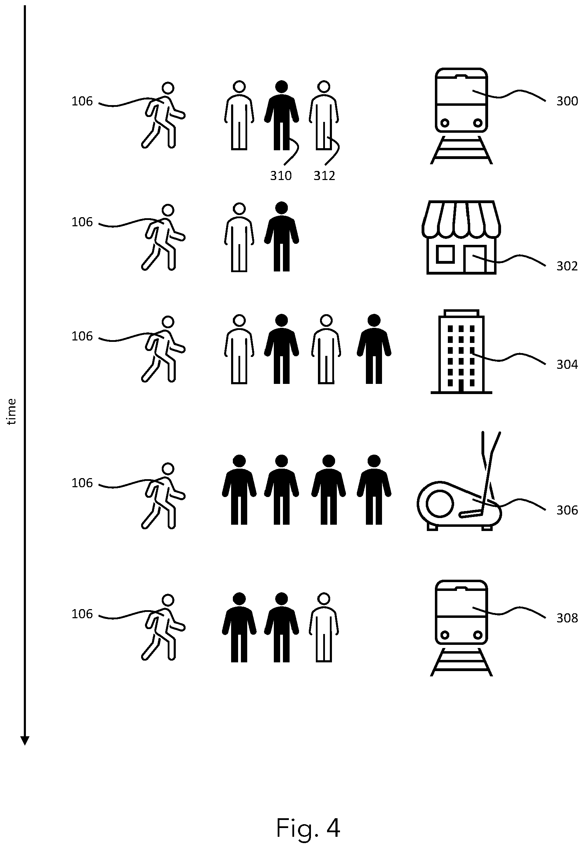

Referring to Figure 4, the user 106 is shown thought a typical day. The user 106 catches a train 300 to work, stops at a coffee shop 302, attends the office 304, goes to the gym 306 after work and catches a train 308 home. During the day, he passes within the vicinity of other people. Some of those people (labelled as active users 310) utilise the same fitness tracking software, use the same metric and have agreed to share that metric with other uses for comparative purposes. Other people (312) do not.

When the user 106 is near to another active user 310, their respective devices communicate with each other using wireless communication in the form of an 'encounter'. The fitness programme on the devices 106 (i.e. an app) is configured to transmit the user's combined fitness score to the other user. No further personal information is shared. The fitness scores are transmitted both ways (i.e. shared with each other). In this embodiment, Bluetooth^{™} will be used to detect the other user, and Wi-Fi or cellular communication (4G / 5G) will be used to share the data. Other methods of wireless communication and data transfer including any combination of Bluetooth^{™}, Wi-Fi, mobile network services, location trackers (GPS) and or NFC (near field communication) are envisaged.

The user 106 is then able to view how he or she compared to other users they have encountered throughout a predetermined time period. This provides them with a useful indication of how they compare to their peers, given that the person 106 is likely to encounter people of similar interests and fitness levels throughout his or her day.

### Variations

Instead of the telemetry devices and the user device being separate, it is possible for them to be integrated. For example, a sufficiently sophisticated smartwatch may carry out some or all of the functionality of the separate user device, and may have the ability for cellular and / or Wi-Fi^{™} communication with the cloud server 108 without an intervening separate device.

## Claims

**1.** A computer-implemented method comprising the steps of:
collecting a first type of health data over *n* days;
collecting a second type of health data over *m* days;
after *n* days:
calculating the average of the first type of health data and normalising the average to produce a first sub-score;
updating an aggregate health score with the first sub-score;
after *m* days:
calculating the average of the second type of health data and normalising the average to produce a second sub-score;
updating the aggregate health score with the second sub-score;
repeating the above steps over a plurality of cycles;
varying n and m between at least two cycles.

**2.** A computer-implemented method according to claim 1, wherein n and m are varied at each cycle.

**3.** A computer-implemented method according to claim 1 or 2, wherein n and m are random integers.

**4.** A computer-implemented method according to claim 3, in which n and m are provided with at least one of an upper and lower limit.

**5.** A computer-implemented method according to any preceding claim, wherein the first and second types of health data are selected from:
a) energy consumed;
b) active time;
c) step count; and,
d) power output.

**6.** A computer-implemented method according to any preceding claim comprising the steps of:
collecting a third type of health data over o days;
calculating the average of the third type of health data and normalising the average to produce a third sub-score;
updating the aggregate health score with the third sub-score;
repeating the above steps over a plurality of cycles;
varying o between at least two cycles.

**7.** A computer-implemented method according to claim 6 comprising the steps of:
collecting a fourth type of health data over *p* days;
calculating the average of the third type of health data and normalising the average to produce a fourth sub-score;
updating the aggregate health score with the fourth sub-score;
repeating the above steps over a plurality of cycles;
varying p between at least two cycles.

**8.** A system for determining a user health score comprising:
a first fitness sensor configured to collect a first type of health data;
a second fitness sensor configured to collect a second type of health data;
a computer configured for communication with the first and second fitness sensors, the computer comprising software configured to implement the method of any preceding claim.

**9.** A system for determining a user health score according to claim 8, wherein the first and second fitness sensors are selected from:
a) energy consumption sensor;
b) active timer;
c) step counter; and,
d) power output meter.

**10.** A system for determining a user health score according to claim 9, wherein the power output meter is comprised within an item of weight training equipment.

**11.** A computer-implemented method comprising the steps of:
providing a first user device associated with a first user, the first user device storing a first user health score;
providing a second user device associated with a second user, the first user device storing a second user health score;
detecting an encounter with the first user device by the presence of the second user device;
anonymising the second user health score;
sending the second user health score from the second user device to the first user device;
comparing the second user health score with the first user health score on the first user device.

**12.** A computer-implemented method according to claim 11, comprising the step of detecting a plurality of encounters over a predetermined time period to determine a sample, and calculating a rank of the first user health score within the sample.

**13.** A computer-implemented method according to claim 11 or 12, wherein the presence of the second user device in the encounter is determined by distance from the first user device, which distance is detected by wireless communication means.

**13.** A computer-implemented method according to claim 13, wherein wireless communication means is Bluetooth (RTM).
